# EUROPEAN PATENT APPLICATION

(11) **EP 4 129 405 A1**
(43) Date of publication of application: **08.02.2023**
(21) Application number: 21782101.6
(22) Date of filing: 26.03.2021
(51) Int. Cl.: A61P 25/00, A61P 43/00, A23L 33/12, A61K 31/202

(54) **COMPOSITION FOR IMPROVING FRIENDLINESS AND/OR EMPATHETIC CAPACITY**

(30) Priority: 01.04.2020 JP 2020065996
(71) Applicant: Suntory Holdings Limited, Osaka 530-8203 (JP)
(72) Inventor: TOKUDA, Hisanori, Soraku-gun, Kyoto 619-0284 (JP); TAKASHIRO, Mika, Soraku-gun, Kyoto 619-0284 (JP); SUEYASU, Toshiaki, Soraku-gun, Kyoto 619-0284 (JP)
(74) Representative: Vossius & Partner Patentanwälte Rechtsanwälte mbB
(86) International application number: PCT/JP2021/012999
(87) International publication number: WO 2021/200698

(57) **Abstract**

The present invention aims to provide a composition for improving friendliness and/or empathy, which can improve friendliness and/or empathy, and a method of improving friendliness and/or empathy. The present invention relates to, for example, a composition for improving friendliness and/or empathy, the composition containing at least one selected from the group consisting of a long-chain polyunsaturated fatty acid having a carbon number of 20 or more and a derivative thereof.

## Description

### TECHNICAL FIELD

The present invention relates to a composition for improving friendliness and/or empathy. More specifically, the present invention relates to a composition for improving friendliness and/or empathy, the composition containing at least one selected from the group consisting of a long-chain polyunsaturated fatty acid and a derivative thereof. The present invention also relates to a method of improving friendliness and/or empathy. The present invention also relates to, for example, use of at least one compound selected from the group consisting of a long-chain polyunsaturated fatty acid having a carbon number of 20 or more and a derivative thereof for improving friendliness and/or empathy.

### BACKGROUND ART

Every person is involved in the society to which he or she belongs to throughout his or her daily life. Thus, maintaining and improving sociality improve people's quality of life (QOL) and lead to creation of a fulfilling life. Presumably, good sociality, such as building a relationship with others and increasing the frequency of contact with an outside environment, is affected by one's friendliness and empathy to others.

In fact, epidemiologic studies have reported that empathy has a positive impact on sociality (Non-Patent Literature 1), suggesting that improved friendliness and empathy to others lead to improved sociality.

Such friendliness and empathy are abilities different from attention, memory, executive function, and the like, and are considered to be a type of brain function. The brain is an organ composed of about 60% lipids, excluding water. Long-chain polyunsaturated fatty acids constituting cell membranes (phospholipids) of brain cells are mainly arachidonic acid and docosahexaenoic acid. However, these arachidonic acid and docosahexaenoic acid cannot be synthesized de *novo* in animal bodies, and are thus required to be ingested directly or indirectly (linoleic acid and α-linolenic acid which are precursors of arachidonic acid and docosahexaenoic acid) through food.

For example, Patent Literature 1 discloses a composition having an action that prevents or ameliorates diseases resulting from a decline in brain function, in which the composition contains, an active ingredient, arachidonic acid and/or a compound containing arachidonic acid as its constituent fatty acid. However, while the composition exhibits effects of reducing a decline in memory and learning ability, a decline in cognitive ability, emotional disorders (e.g., depression), intellectual disabilities (e.g., dementia, specifically, Alzheimer's disease and cerebrovascular dementia), no effect on friendliness and empathy has been demonstrated.

Non-Patent Literature 2 discloses an effect of oral nutritional supplements containing n-3 polyunsaturated fatty acids on the quality of life (QOL) and functional status in lung cancer patients during multimodality treatment (DISCUSSION). Non-Patent Literature 3 discloses that a long-chain polyunsaturated fatty acid that is the richest fatty acid in the brain is essential in growth and development of the brain and retina, and ingestion of a fish oil supplement (containing docosahexaenoic acid and eicosapentaenoic acid) by pregnant women during the perinatal period is useful for neurodevelopment in the domain of communications of 4-month-old infants. Non-Patent Literature 4 discloses that while autism spectrum disorder (ASD) is a neurodevelopmental disorder with declined functional connectivity of cortex associated with social cognition, arachidonic acid (ARA) and docosahexaenoic acid (DHA) possibly play important roles in the maturation of the brain network, and ingestion of DHA and ARA may help individuals with ASD reduce their barriers to social interactions. Non-Patent Literature 5 suggests that a long-chain omega-3 fatty acid improves cognitive depressive symptoms.

However, these literatures nowhere demonstrate the effect on friendliness and empathy.

As described above, long-chain polyunsaturated fatty acids have been studied, but effects of ingestion of long-chain polyunsaturated fatty acids on friendliness and empathy are yet to be demonstrated and largely remain unknown.

Non-Patent Literature 6 suggests improving social function by exercise, but nowhere demonstrates an effect of exercise on friendliness and empathy, and thus, the effect remains unknown.

### CITATION LIST

### - Patent Literature

Patent Literature 1: JP 2012-036214 A

### - Non-Patent Literature

Non-Patent Literature 1: Aging & Mental Health Vol. 12, No. 4, July 2008, 499-503, "Empathy and social functioning in late adulthood"
Non-Patent Literature 2: European Journal of Clinical Nutrition (2012) 66, 399-404, "Oral nutritional supplements containing n-3 polyunsaturated fatty acids affect quality of life and functional status in lung cancer patients during multimodality treatment: an RCT"
Non-Patent Literature 3: Eur J Nutr (2018) 57:2387-2397, "The effect of perinatal fish oil supplementation on neurodevelopment and growth of infants: a randomized controlled trial"
Non-Patent Literature 4: Journal of Clinical Psychopharmacology, Volume 32, Number 2, April 2012, "Effects of Large Doses of Arachidonic Acid Added to Docosahexaenoic Acid on Social Impairment in Individuals With Autism Spectrum Disorders"
Non-Patent Literature 5: JACC: HEART FAILURE VOL. 6, NO. 10, 2018, "Long-Chain Omega-3 Fatty Acid Supplements in Depressed Heart Failure Patients"
Non-Patent Literature 6: Australian Journal of Physiotherapy 2005, Vol. 51, "Effects of a water-based program on women 65 years and over: A randomized controlled trial"

### SUMMARY OF INVENTION

### - Technical Problem

The present invention aims to provide a composition for improving friendliness and/or empathy, which can improve friendliness and/or empathy that particularly affects relationships with people in social life. The present invention also aims to provide a method of improving friendliness and/or empathy.

### - Solution to Problem

Specifically, the present invention relates to the following compositions, method of improving friendliness and/or empathy, and the like.
(1) A composition for improving friendliness and/or empathy, containing at least one selected from the group consisting of a long-chain polyunsaturated fatty acid having a carbon number of 20 or more and a derivative thereof.
(2) The composition according to (1) above for use in improving sociality by improving friendliness and/or empathy.
(3) The composition according to (1) or (2) above, wherein the long-chain polyunsaturated fatty acid is at least one selected from the group consisting of arachidonic acid, docosahexaenoic acid, and eicosapentaenoic acid.
(4) The composition according to (3) above, wherein the long-chain polyunsaturated fatty acid includes arachidonic acid, docosahexaenoic acid, and eicosapentaenoic acid.
(5) The composition according to (4) above, wherein a weight ratio of the docosahexaenoic acid to the arachidonic acid is 0.01:1 to 500:1 and a weight ratio of the eicosapentaenoic acid to the arachidonic acid is 0.01:1 to 500:1.
(6) The composition according to any one of (1) to (5) above, wherein the improvement in friendliness and/or empathy is assessed by POMS 2^{™}.
(7) The composition according to (6) above, wherein the improvement in friendliness and/or empathy is assessed by a friendliness scale of POMS 2^{™}.
(8) The composition according to any one of (1) to (7) above, wherein the composition is used in combination with an exercise.
(9) The composition according to any one of (1) to (8) above, wherein the composition is a food composition or a pharmaceutical composition.
(10) The composition according to (9) above, wherein the composition is a food composition, and the food composition is a functional food, a dietary supplement, a food for specified health uses, or a food for the elderly.
(11) A method of improving friendliness and/or empathy, the method including: administering at least one compound selected from the group consisting of a long-chain polyunsaturated fatty acid having a carbon number of 20 or more and a derivative thereof.
(12) Use of at least one compound selected from the group consisting of a long-chain polyunsaturated fatty acid having a carbon number of 20 or more and a derivative thereof for improving friendliness and/or empathy.

### - Advantageous Effects of Invention

The present invention can provide a composition for improving friendliness and/or empathy, which can improve friendliness and/or empathy, the composition containing at least one selected from the group consisting of a long-chain polyunsaturated fatty acid and a derivative thereof as an active ingredient.

### DESCRIPTION OF EMBODIMENTS

The composition of the present invention is a composition for improving friendliness and/or empathy, the composition containing at least one selected from the group consisting of a long-chain polyunsaturated fatty acid (hereinafter also referred to as "LCPUFA") having a carbon number of 20 or more and a derivative thereof. The composition for improving friendliness and/or empathy of the present invention contains at least one selected from the group consisting of a long-chain polyunsaturated fatty acid having a carbon number of 20 or more and a derivative thereof as an active ingredient.

Here, examples of the derivative of the long-chain polyunsaturated fatty acid (LCPUFA) having a carbon number of 20 or more include esters and vinyl ethers of LCPUFA having a carbon number of 20 or more, including a compound having a structure to which a LCPUFA having a carbon number of 20 or more is bonded and a compound capable of separating (generating) a LCPUFA having a carbon number of 20 or more by hydrolysis.

The long-chain polyunsaturated fatty acid for use in the composition of the present invention is not limited as long as it is a fatty acid having a carbon number of 20 or more and having two or more carbon unsaturated bonds. Specifically, examples thereof include at least one selected from the group consisting of eicosadienoic acid, eicosatrienoic acid, eicosatetraenoic acid, eicosapentaenoic acid, docosadienoic acid, docosatrienoic acid, docosatetraenoic acid, docosapentaenoic acid, docosahexaenoic acid, tetracosadienoic acid, tetracosatrienoic acid, tetracosatetraenoic acid, tetracosapentaenoic acid, tetracosahexaenoic acid, and arachidonic acid. The long-chain polyunsaturated fatty acid is preferably at least one selected from the group consisting of arachidonic acid, docosahexaenoic acid, and eicosapentaenoic acid.

Usable examples of the derivative of the long-chain polyunsaturated fatty acid having a carbon number of 20 or more include all the compounds having a structure to which a long-chain polyunsaturated fatty acid having a carbon number of 20 or more is bonded and all the compounds capable of separating a long-chain polyunsaturated fatty acid having a carbon number of 20 or more by hydrolysis. For example, when the long-chain polyunsaturated fatty acid having a carbon number of 20 or more is at least one of arachidonic acid, docosahexaenoic acid, or eicosapentaenoic acid, usable examples of the derivative of the long-chain polyunsaturated fatty acid include all the compounds having a structure to which arachidonic acid, docosahexaenoic acid, and/or eicosapentaenoic acid is bonded and all the compounds capable of separating arachidonic acid, docosahexaenoic acid and/or eicosapentaenoic acid by hydrolysis.

Examples of the compounds having a structure to which a long-chain polyunsaturated fatty acid having a carbon number of 20 or more is bonded and the compounds capable of separating a long-chain polyunsaturated fatty acid having a carbon number of 20 or more by hydrolysis include salts, such as calcium salt and sodium salt, of the long-chain polyunsaturated fatty acids having a carbon number of 20 or more, and alcohol esters of the long-chain polyunsaturated fatty acid having a carbon number of 20 or more, such as arachidonic acid methyl ester, docosahexaenoic acid ethyl ester, and eicosapentaenoic acid ethyl ester. Usable examples of the derivative include triglycerides, diglycerides, monoglycerides, phospholipids, and glycolipids in which one or more or all of the constituent fatty acids are the long-chain polyunsaturated fatty acids having a carbon number of 20 or more.

When the at least one selected from the group consisting of a long-chain polyunsaturated fatty acid having a carbon number of 20 or more and a derivative thereof is at least one selected from the group consisting of arachidonic acid, docosahexaenoic acid, eicosapentaenoic acid, and derivatives thereof, the long-chain polyunsaturated fatty acid having a carbon number of 20 or more is preferably in a triglyceride or phospholipid form, particularly preferably a derivative in a triglyceride form, in terms of suitability to food.

Thus, in the present invention, the derivative of the long-chain polyunsaturated fatty acid having a carbon number of 20 or more can be a triglyceride in which one or more or all of the constituent fatty acids are the long-chain polyunsaturated fatty acids having a carbon number of 20 or more (a triglyceride containing one or more long-chain polyunsaturated fatty acids having a carbon number of 20 or more). A preferred form of the triglyceride containing one or more long-chain polyunsaturated fatty acids having a carbon number of 20 or more when applied to food is an oil or fat (triglyceride) in which the proportion of the long-chain polyunsaturated fatty acid(s) having a carbon number of 20 or more of all the fatty acids constituting the triglyceride is 5 wt% (w/w%) or more, preferably 10 wt% or more, more preferably 20 wt% or more, most preferably 30 wt% or more. The origins of the long-chain polyunsaturated fatty acid having a carbon number of 20 or more and a derivative thereof are not limited. Any product obtained, for example, by culturing microorganisms capable of producing an oil or fat (triglyceride) containing arachidonic acid can be used as the derivative of arachidonic acid in the present invention, for example.

For example, any fish oil or concentrated fish oil of blue-backed fish or the like containing an oil or fat (triglyceride) containing docosahexaenoic acid and/or eicosapentaenoic acid can be used as the derivative of docosahexaenoic acid and/or eicosapentaenoic acid.

When the composition contains multiple long-chain polyunsaturated fatty acids having a carbon number of 20 or more, a preferred form of the triglyceride containing the long-chain polyunsaturated fatty acids having a carbon number of 20 or more when applied to food is an oil or fat (triglyceride) in which the total proportion of the long-chain polyunsaturated fatty acids having a carbon number of 20 or more of all the fatty acids constituting the triglyceride is 5 wt (w/w) % or more, preferably 10 wt% or more, more preferably 20 wt% or more, still more preferably 30 wt% or more.

Further, more preferably, the composition of the present invention contains arachidonic acid, docosahexaenoic acid, and eicosapentaenoic acid as the long-chain polyunsaturated fatty acids having a carbon number of 20 or more.

When the composition contains the arachidonic acid, the docosahexaenoic acid, and the eicosapentaenoic acid as the long-chain polyunsaturated fatty acids having a carbon number of 20 or more, preferably, the weight ratio of the docosahexaenoic acid to the arachidonic acid is 0.01:1 to 500:1 and the weight ratio of the eicosapentaenoic acid to the arachidonic acid is 0.01:1 to 500:1. The weight ratio of each of the docosahexaenoic acid and the eicosapentaenoic acid to the arachidonic acid is more preferably 0.05:1 to 100:1, still more preferably 0.1:1 to 50:1.

The composition of the present invention is a composition for improving friendliness and/or empathy, and preferably, the improvement in friendliness and/or empathy is assessed by POMS 2 (Profile of Mood States 2nd Edition).

POMS 2 is an unregistered trademark of Multi-Health Systems Inc. Psychological tests may have different test methods depending on the target population such as adults or youth, or may have different numbers of questions (full version or short version) at the time of testing. The test method and the number of questions can be appropriately selected depending on the target conditions.

Specifically, POMS 2 measures factors including "Tension-Anxiety; TA", "Depression-Dejection; D", "Anger-Hostility; AH", "Vigor Activity; VA", "Fatigue-Inertia; FI", "Confusion-Bewilderment; CB", and "Friendliness; F".

POMS 2 includes "Profile of Mood States 2nd Edition-Adult (POMS 2-A)" and "Profile of Mood States 2nd Edition-Adult Short (POMS 2-A short)" (originally developed by Heuchert, J. P. et al.; Japanese version: Kazuhito Yokoyama (translation supervisor), Kanokoshobo, 2015).

Assessment of mood using "Profile of Mood States 2nd Edition-Adult", "Profile of Mood States 2nd Edition-Adult Short", "Profile of Mood States 2nd Edition-Youth", and "Profile of Mood States 2nd Edition-Youth Short" is one of the tests for objectively measuring the psychological state of the measurement target using a questionnaire. The validity of assessment by POMS 2 is confirmed worldwide. POMS 2 is used to understand mood change due to pharmaceutical products, food, exercise, relaxation, and the like.

In the case of the composition for improving friendliness and/or empathy of the present invention, the improvement in friendliness and/or empathy is preferably measured using "Profile of Mood States 2nd Edition-Adult" or "Profile of Mood States 2nd Edition-Adult Short", and is more preferably measured using "Profile of Mood States 2nd Edition-Adult".

"Profile of Mood States 2nd Edition-Adult" includes the following question items for measuring "friendliness" factors: (1) I enjoy socializing; (2) I have compassion for others; (3) I feel sympathy for others; (4) I have a feeling of being useful to others; (5) I can treat people warmly; (6) I trust others; and the like. Among the (1) to (6) question items, the question items (1) to (4) relate to empathy, which allows for measurement of friendliness and empathy.

The "friendliness" factors of POMS 2 indicate the state of mood with trust and compassion for others. Herein, the "improvement in friendliness" refers to an improvement in the value of "friendliness" assessed by POMS 2. Herein, the "improvement in empathy" also refers to an improvement in the value of response to at least one selected from (1) I enjoy socializing; (2) I have compassion for others; (3) I feel sympathy for others; and (4) I have a feeling of being useful to others.

Preferably, the composition of the present invention is used for improving sociality by improving friendliness and/or empathy. Generally, the sociality is described as a disposition to create a group and live in the group, or one's attitude toward life to socialize with the surrounding people (sociability). Herein, the "sociality" refers to one's attitude toward life to socialize with the surrounding people (sociability). Specifically, an increase in the frequency of social activities such as visiting others, participating in local activities such as club activities, and meeting people for conversation or dining out is referred to as "improvement in sociality". The improvement in sociality includes maintenance of sociality and prevention of decline in sociality.

According to recent studies by Non-Patent Literature 1 described above and the like, epidemiologic studies have reported that empathy has a positive effect on sociality. Thus, the improvement in friendliness and/or empathy leads to an improvement in sociality.

The composition of the present invention is used for improving friendliness and/or empathy, and is preferably used in combination with an exercise. The composition, when used in combination with an exercise, can further exhibit the effect of improving friendliness and/or empathy. The term "exercise" used herein may refer to any of the following: aerobic exercises such as jogging, walking, calisthenics, and aerobics; resistance exercise for increasing the muscle mass and muscle strength, such as squat, bench press, side raise, rowing, calf raise, and sit-up; and stretching exercise. More preferably, the composition of the present invention is used in combination with two or more of aerobic exercises, resistance exercise, and stretching exercise. Still more preferably, the composition is used in combination with aerobic exercise and resistance exercise. For example, the composition of the present invention may be used in combination with stretching exercise and at least one of aerobic exercise or resistance exercise, and may be used with stretching exercise, aerobic exercise, and resistance exercise. In this case, stretching exercise may be performed before or after, or before and after aerobic exercise and/or resistance exercise.

The frequency and duration of the exercise may be, for example, one or more days in one week, five minutes or more (preferably 30 minutes or more) per time per day, preferably two days or more in one week, five minutes or more (preferably 30 minutes or more) per time per day, more preferably three days or more in one week, five minutes or more (preferably 30 minutes or more) per time per day, still more preferably five days or more in one week, five minutes or more (preferably 30 minutes or more) per time per day. The frequency and/or duration of the exercise may be determined depending on the subject, type of exercise, and/or combination of exercises. For example, aerobic exercise may be performed two to five days in one week, about 10 to 60 minutes per time per day; resistance exercise may be performed one to three days in one week, about 10 to 60 minutes per time per day; and stretching exercise may be performed one to seven days in one week, about five to thirty minutes per time per day.

The intensity of exercise to be combined with the composition of the present invention is preferably 2.0 METs (e.g., walking/very slow/17151) or more, more preferably 2.3 METs (e.g., stretching/mild/02101) or more, still more preferably 3.5 METs (e.g., resistance weight training/02054) or more. Preferably, the exercise load is determined depending on the subject (for the detail of the intensity of exercise, see "Revised Physical Activity METS Chart (National Institute of Health and Nutrition 2012)").

Preferably, the composition of the present invention is a food composition or a pharmaceutical composition.

Examples of the food composition include general foods, functional foods, dietary supplements, foods for specified health uses, formulas for premature infants, formulas for infants, foods for infants, foods for pregnant women, and foods for the elderly. Of these, the food composition of the present invention is preferably a functional food, a dietary supplement, a food for specified health uses, or a food for the elderly.

Preferred foods are fat- or oil-containing foods. Examples thereof include natural foods inherently containing fat or oil such as meat, fish, or nuts; foods to which fat or oil is added at the time of cooking, such as soups; foods using fat or oil as a heating medium, such as doughnuts; fat or oil foods such as butter; processed foods to which fat or oil is added at the time of processing, such as cookies; and foods to which fat or oil is sprayed or applied at the time of finish processing, such as hard biscuits. Further, the long-chain polyunsaturated fatty acid having a carbon number of 20 or more, which is an active ingredient of the present invention, can be added to fat- and oil-free foods such as agricultural foods, fermented foods, livestock foods, marine foods, or beverages.

Further, the composition of the present invention may be in the form of a functional food, a pharmaceutical product, or a quasi-pharmaceutical product. For example, the composition may be a processed product. Examples thereof include enteral nutrients, powders, granules, troches, liquid medicines for internal use, suspensions, emulsions, and syrups.

The food composition of the present invention contains at least one selected from the group consisting of a long-chain polyunsaturated fatty acid having a carbon number of 20 or more and a derivative thereof. Preferably, the food composition contains at least one selected from arachidonic acid, docosahexaenoic acid, eicosapentaenoic acid, and a compound containing any of these acids as its constituent fatty acid(s). The food composition can be labelled with a function claim based on the action that improves friendliness and/or empathy or an action based on such an action.

Examples of such function claims include maintaining and improving cognitive empathy, maintaining and improving emotional empathy, enjoying socializing, having compassion for others, feeling sympathy for others, feeling of being useful to others, being able to treat people warmly, trusting others, maintaining and improving kindness, maintaining and increasing willingness to participate in society, maintaining and improving sociality, maintaining and improving communication skills, maintaining and improving active listening skills, and maintaining and improving conversation skills.

The composition of the present invention contains at least one selected from the group consisting of a long-chain polyunsaturated fatty acid having a carbon number of 20 or more and a derivative thereof as an active ingredient. The composition preferably contains at least one selected from arachidonic acid, docosahexaenoic acid, eicosapentaenoic acid, and a compound containing any of these acids as its constituent fatty acid(s) as the active ingredient, and may also contain various carriers and additives generally used in food or beverages, pharmaceutical products, or quasi-pharmaceutical products. In particular, the composition preferably contains an antioxidant for the purpose of preventing oxidation of the active ingredients. The antioxidant may be a natural antioxidant and/or a synthetic antioxidant. Examples of natural antioxidants include tocopherols, flavone derivatives, phyllodulcins, kojic acid, gallic acid derivatives, catechins, fukiic acid, gossypol, pyrazine derivative, sesamol, guaiacol, guaiacol acid, p-coumaric acid, nordihydroguaiaretic acid, sterols, terpenes, nucleobases, carotenoids, and lignans. Examples of synthetic antioxidants include ascorbic acid palmitate, ascorbic acid stearate, butylated hydroxyanisole (BHA), butylated hydroxytoluene (BHT), mono-t-butylhydroquinone (TBHQ), and 4-hydroxymethyl-2,6-di-t-butylphenol (HMBP).

Examples of the tocopherols include α-tocopherol, β-tocopherol, γ-tocopherol, tocopherols, and tocopherol esters (e.g., tocopherol acetate). Examples of the carotenoids include β-carotene, canthaxanthin, and astaxanthin.

Examples of the carriers include various types of carriers, extenders, diluents, bulking agents, dispersants, excipients, binder solvents (e.g., water, ethanol, and vegetable oils), dissolution aids, buffers, dissolution accelerators, gelling agents, suspending agents, wheat flour, rice flour, starch, cornstarch, polysaccharide, milk protein, collagen, rice oil, and lecithin. Non-limiting examples of the additives include vitamins, sweeteners, organic acids, colorants, flavoring or masking agents, anti-wetting agents, fibers, electrolytes, minerals, nutrients, antioxidants, preservatives, aromatic agents, wetting agents, natural food extracts, and vegetable extracts.

The intake of the composition of the present invention per day in terms of the intake of the long-chain polyunsaturated fatty acid having a carbon number of 20 or more is preferably 0.001 g to 20 g, more preferably 0.01 g to 10 g, still more preferably 0.05 g to 5 g, particularly preferably 0.1 g to 2 g for an adult (e.g., 60 kg body weight) per day. In particular, the total intake of arachidonic acid, docosahexaenoic acid, and eicosapentaenoic acid is preferably in the above range.

When the active ingredient of the present invention is actually used in a food or beverage, the absolute amount of a long-chain polyunsaturated fatty acid having a carbon number of 20 or more to be added to the food or beverage is also important. Yet, the absolute amount to be added to the food or beverage also varies depending on the intake of the food or beverage to which the active ingredient is added. Thus, when a triglyceride containing a triglyceride in which one or more or all of the constituent fatty acids are the long-chain polyunsaturated fatty acids having a carbon number of 20 or more is added to food, preferably, the triglyceride is added such that its amount in terms of the long-chain polyunsaturated fatty acid having a carbon number of 20 or more is preferably 0.001 wt% or more, more preferably 0.01 wt% or more, still more preferably 0.1 wt% or more. The long-chain polyunsaturated fatty acid having a carbon number of 20 or more is added to an amount of preferably 90 wt% or less, more preferably 85 wt% or less, still more preferably 80 wt% or less.

For use as a pharmaceutical product, the composition of the present invention can be produced in accordance with a conventional method in the pharmaceutical technology field, for example, a method described in Japanese Pharmacopoeia or a method similar thereto.

For use as a pharmaceutical product, the ingredient(s) in the composition of the present invention may be contained in any proportion as long as the object of the present invention is achieved. The ingredient(s) can be used in any appropriate proportion.

For use as a pharmaceutical product, the composition of the present invention is preferably administered in dosage unit form, particularly, in the form of oral administration.

The dosage of the composition of the present invention varies depending on age, body weight, symptoms, number of doses, and the like. For example, the dosage of the composition to an adult (about 60 kg) per day in terms of the long-chain polyunsaturated fatty acid having a carbon number of 20 or more is usually 0.001 g to 20 g. Preferably, the composition is administered in one to three portions per day with a dose of preferably about 0.01 g to 10 g, more preferably about 0.05 g to 5 g, still more preferably about 0.1 g to 2 g.

A food or beverage having an action that improves friendliness and/or empathy may be produced, for example, by a method in which a long-chain polyunsaturated fatty acid having a carbon number of 20 or more and/or a derivative thereof is added alone to a food or beverage ingredient substantially free of a long-chain polyunsaturated fatty acid having a carbon number of 20 or more. The food or beverage ingredient substantially free of a long-chain polyunsaturated fatty acid having a carbon number of 20 or more includes a food or beverage which contains a long-chain polyunsaturated fatty acid having a carbon number of 20 or more but in which the intake per day in terms of the long-chain polyunsaturated fatty acid having a carbon number of 20 or more is short of 0.001 g.

A subject (administration subject) to which the composition of the present invention is applicable is not limited. For example, the composition is applicable to humans and non-human mammals, with humans being preferred. The subject may be, for example, one wanting or needing improvement in friendliness and/or empathy, one wanting or needing improvement in sociality by improving friendliness and/or empathy, or the like. Examples of such a subject include healthy adults with an apparent decline in friendliness and/or empathy and healthy middle-aged and older people with an apparent decline in friendliness and/or empathy. Herein, the middle-aged and older people may be adults who are 40 years old and older, for example. In recent years, countries such as Japan have seen an increase in the elderly proportion, and extension of healthy life span and improvement in quality of daily life (QOL) have been challenges. For example, improvement in sociality among the elderly is likely to contribute to extension of healthy life span and improvement in QOL of the elderly. Thus, healthy elderly are preferred subjects (administration subjects) of the composition of the present invention. Herein, the elderly may be adults who are 60 years old and older.

The present invention also encompasses the following uses and methods:
use of at least one compound selected from the group consisting of a long-chain polyunsaturated fatty acid having a carbon number of 20 or more and a derivative thereof for improving friendliness and/or empathy, or use of a composition for improving friendliness and/or empathy, the composition containing the compound;
use of at least one compound selected from the group consisting of a long-chain polyunsaturated fatty acid having a carbon number of 20 or more and a derivative thereof for improving sociality by improving friendliness and/or empathy, or use of a composition for improving friendliness and/or empathy, the composition containing the compound;
a method of improving friendliness and/or empathy, the method including administering to a subject the at least one compound selected from the group consisting of a long-chain polyunsaturated fatty acid having a carbon number of 20 or more and a derivative thereof, or the composition for improving friendliness and/or empathy, the composition containing the compound; and
a method of improving sociality by improving friendliness and/or empathy, the method including administering to a subject the at least one compound selected from the group consisting of a long-chain polyunsaturated fatty acid having a carbon number of 20 or more and a derivative thereof, or the composition for improving friendliness and/or empathy, the composition containing the compound.

In the uses and methods, the long-chain polyunsaturated fatty acid having a carbon number of 20 or more and a derivative thereof, the composition of the present invention, the administration subject, and preferred embodiments of these are the same as those of the composition of the present invention described above. In the uses and methods, the composition of the present invention can be used in the form of a food or beverage, a pharmaceutical product, or the like.

The uses may be therapeutic or non-therapeutic.

The methods may be therapeutic or non-therapeutic.

The "non-therapeutic" is a concept that does not include medical activities, i.e., a concept that does not include methods of surgery, therapy, or diagnosis of humans.

All academic literature and patent literature described herein are incorporated herein by reference.

### EXAMPLES

The present invention is described more specifically below with reference to examples, but the present invention is not limited to these examples.

### (Example 1, Comparative Examples 1 and 2)

### <Production of test food>

An arachidonic acid-containing edible oil or fat (triglyceride) obtained by culturing microorganisms of the genus *Mortierella* with aeration and stirring was combined with an oil or fat (triglyceride) containing DHA and EPA collected from fish to produce a fat or oil containing a long-chain polyunsaturated fatty acid (LCPUFA) in which arachidonic acid (ARA), docosahexaenoic acid (DHA), and eicosapentaenoic acid (EPA) were contained in a weight ratio of 6:15:5.

Gelatin (100 parts by weight) and food additive glycerol (30 parts by weight) were dissolved in water at 50°C to 60°C, whereby a base material for soft capsule coating was prepared. Then, capsules were formed and dried by a common method using the LCPUFA-containing fat or oil to produce soft capsules containing 180 mg of content per capsule (size of one capsule: major axis 10.7 mm × minor axis 7.0 mm), and an LCPUFA-containing food was produced in which the amount of ARA was 120 mg, the amount of DHA was 300 mg, and the amount of EPA was 100 mg per six capsules. As a control food for clinical trials, soft capsules containing refined olive oil were produced. A set of six capsules of the LCPUFA-containing food and a set of six capsules of the control food were individually packaged in aluminum pouches to prevent deterioration such as oxidation, and the number of sets required for clinical trials was prepared.

### <Clinical trials>

The consent was informed to those participating in the trials, and healthy elderly of 60 to 79 years old (without a habit of exercise) who agreed to the consent were subjected to a placebo-controlled, single-blind, parallel-group study. The subjects were divided into the following three groups: no exercise + control food (Comparative Example 1), exercise + control food (Comparative Example 2), and exercise + LCPUFA-containing food (Example 1). Each group was subjected to intervention with exercise and intake of the test food for 24 weeks. In the exercise group, the subjects performed a resistance exercise (three to five sets of squat, bench press, side raise, rowing, calf raise, and sit-up with about 10 RM each) for 30 minutes per day, two days a week, and an aerobic exercise (walking, 12 to 13 RPE) for 30 minutes per day, three days a week. Each type of exercise included stretching for warming up before the main exercise and stretching for cooling down after the main exercise. The no-exercise group was not subjected to active intervention with exercise, and was only allowed to perform daily activities. Six capsules of the test food (control food or LCPUFA-containing food) per day were ingested with a sufficient amount of water or warm water. Mood states were assessed before and after the intervention using Profile of Mood States 2 (POMS 2) with worldwide recognition for its validity, and the impact of the intervention on mood states was assessed. POMS 2 includes seven sub-scales. In particular, the "friendliness" is assessed with questions including "I enjoy socializing", "I have compassion for others", "I feel sympathy for others", "I have a feeling of being useful to others", "I can treat people warmly", and "I trust others", which allows for assessment of friendliness and empathy, for example.

### <Analysis results>

The numbers of the analysis subjects who completed the intervention were as follows: no exercise + control food group: 28 subjects; exercise + control food group: 27 subjects; and exercise + LCPUFA-containing food group: 21 subjects. Table 1 shows the test results (T scores) on the "friendliness" by POMS 2. The test results are scores (T scores) calculated by comparing the total raw points for the questions regarding the "friendliness" of POMS 2 against the conversion table standardized by sex and age. The average is 50 points. The mean ± standard error for each group before the intervention was as follows: no exercise + control food group: 50.9 ± 1.8 points; exercise + control food group: 51.8 ± 2.4 points; and exercise + LCPUFA-containing food group: 47.7 ± 2.1 points. No significant difference was found among the three groups. The exercise + control food group and the exercise + control food group showed no significant changes before and after the intervention. The exercise showed no significant improving effect on the "friendliness" assessed by POMS 2. In contrast, the exercise + LCPUFA-containing food group showed a significant increase of 6.6 points. As described above, only the group subjected to ingestion of LCPUFA showed an improvement in the index, which confirms the usefulness of LCPUFA ingestion on the friendliness/empathy.

**[Table 1]**

| | Before intervention a) | After interventin b) | Change |
|---|---|---|---|
| No exercise + control food group | 50.9 ± 1.8 | 52.9 ± 1.9 | 2.0 ± 1.8 |
| Exercise + control food group | 51.8 ± 2.4 | 52.6 ± 2.3 | 0.8 ± 2.0 |
| Exercise + LCPUFA-containing food group | 47.7 ± 2.1 | 54.3 ± 2.4** | 6.6 ± 1.7 |

| | | | |
|---|---|---|---|
| The results are expressed as mean ± standard error. (No exercise + control food group: n = 28, exercise + control food group: n = 27, exercise + LCPUFA-containing food: n = 21) a) No significant difference among the three groups (one-way ANOVA) b) A significant difference was found before and after the invention (** p < 0.01, paired t-test) | | | |

## Claims

1. A composition for improving friendliness and/or empathy, comprising:
at least one selected from the group consisting of a long-chain polyunsaturated fatty acid having a carbon number of 20 or more and a derivative thereof.

2. The composition according to claim 1 for use in improving sociality by improving friendliness and/or empathy.

3. The composition according to claim 1 or 2,
wherein the long-chain polyunsaturated fatty acid is at least one selected from the group consisting of arachidonic acid, docosahexaenoic acid, and eicosapentaenoic acid.

4. The composition according to claim 3,
wherein the long-chain polyunsaturated fatty acid includes arachidonic acid, docosahexaenoic acid, and eicosapentaenoic acid.

5. The composition according to claim 4,
wherein a weight ratio of the docosahexaenoic acid to the arachidonic acid is 0.01:1 to 500:1 and a weight ratio of the eicosapentaenoic acid to the arachidonic acid is 0.01:1 to 500:1.

6. The composition according to any one of claims 1 to 5,
wherein the improvement in friendliness and/or empathy is assessed by POMS 2^{™}.

7. The composition according to claim 6,
wherein the improvement in friendliness and/or empathy is assessed by a friendliness scale of POMS 2^{™}.

8. The composition according to any one of claims 1 to 7,
wherein the composition is used in combination with an exercise.

9. The composition according to any one of claims 1 to 8,
wherein the composition is a food composition or a pharmaceutical composition.

10. The composition according to claim 9,
wherein the composition is a food composition, and the food composition is a functional food, a dietary supplement, a food for specified health uses, or a food for the elderly.

11. A method of improving friendliness and/or empathy, the method comprising:
administering at least one compound selected from the group consisting of a long-chain polyunsaturated fatty acid having a carbon number of 20 or more and a derivative thereof.

12. Use of at least one compound selected from the group consisting of a long-chain polyunsaturated fatty acid having a carbon number of 20 or more and a derivative thereof for improving friendliness and/or empathy.
